(19) Europäisches Patentamt
European Patent Office
Office européen des brevets

(11) Veröffentlichungsnummer: **0 062 254**
**A1**

(12) # EUROPÄISCHE PATENTANMELDUNG

(21) Anmeldenummer: **82102540.0**

(22) Anmeldetag: **26.03.82**

(51) Int. Cl.³: **C 07 D 263/58**, C 07 D 277/68, A 01 N 43/74

(30) Priorität: **06.04.81 JP 50562/81**

(43) Veröffentlichungstag der Anmeldung: **13.10.82 Patentblatt 82/41**

(84) Benannte Vertragsstaaten: **BE CH DE FR GB IT LI NL**

(71) Anmelder: **NIHON TOKUSHU NOYAKU SEIZO K.K., No.8, 2-chome, Nihonbashi Muromachi, Chuo-Ku, Tokyo (JP)**

(72) Erfinder: **Kuyama, Shinpei, 3-21-39, Meguro, Meguro-ku Tokyo (JP)**
Erfinder: **Aya, Masahiro, 2-844, Suzuki-cho, Kodaira-shi Tokyo (JP)**
Erfinder: **Saito, Junichi, 3-7-12, Osawa, Mitaka-shi Tokyo (JP)**

(74) Vertreter: **Schumacher, Günter, Dr. et al, c/o Bayer AG Zentralbereich Patente, Marken und Lizenzen, D-5090 Leverkusen 1 Bayerwerk (DE)**

(54) **Substituierte Acetanilide, Verfahren zu ihrer Herstellung und ihre Verwendung als Herbizide.**

(57) Die Erfindung betrifft neue substituierte Acetanilide der allgemeinen Formel

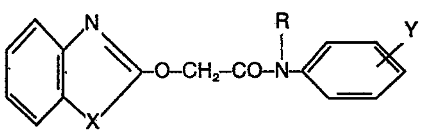

(I)

worin X für Sauerstoff oder Schwefel, Y für $C_{1-4}$-Alkylthio und R für $C_{1-4}$-Alkyl steht, ein Verfahren zu ihrer Herstellung und ihre Verwendung als Herbizide.

EP 0 062 254 A1

0062254

NIHON TOKUSHU NOYAKU SEIZO K.K. /Tokyo /Japan

Bi/Bck
Ia

## Substituierte Acetanilide, Verfahren zu ihrer Herstellung und ihre Verwendung als Herbizide

Die vorliegende Erfindung betrifft substituierte Acetanilid-Verbindungen, ein Verfahren zu deren Herstellung und diese enthaltende Herbizide.

Die substituierten Acetanilid-Verbindungen gemäß der vorliegenden Erfindung besitzen die nachstehende Formel

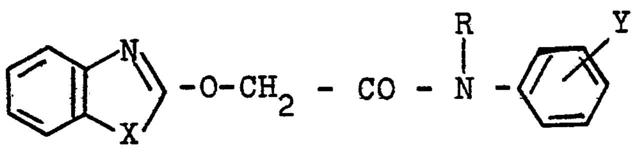

(I)

in der X für ein Sauerstoff-Atom oder ein Schwefel-Atom, Y für eine Alkylthio-Gruppe mit 1 - 4 Kohlenstoff-Atomen und R für eine Alkyl-Gruppe mit 1 - 4 Kohlenstoff-Atomen steht.

Die vorliegende Erfindung betrifft außerdem ein Verfahren zur Herstellung der Verbindungen der vorstehenden Formel (I). Dieses Verfahren zur Herstellung einer substituierten Acetanilid-Verbindung der vorstehenden Formel (I) ist dadurch gekennzeichnet, daß man ein 2-Halogenobenzazol der Formel

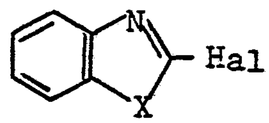

(II)

in der X die vorstehend genannte Bedeutung hat und Hal für ein Halogen-Atom steht, mit einer Verbindung der Formel

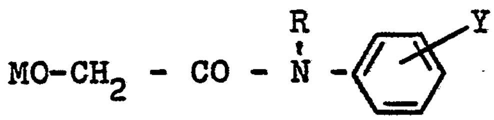

(III)

in der Y und R die vorstehend genannten Bedeutungen haben und M für ein Wasserstoff-Atom oder ein Alkalimetall-Atom steht, zur Reaktion bringt.

Die vorliegende Erfindung betrifft weiterhin Herbizide, die substituierte Acetanilid-Verbindungen der vorstehenden Formel (I) als Wirkstoffe enthalten.

In der JP-OS 154862/1979 (U.S. Serial No. 35361) wird beschrieben, daß substituierte Carbonsäureamide der nachstehenden Formel herbizide Wirkung besitzen:

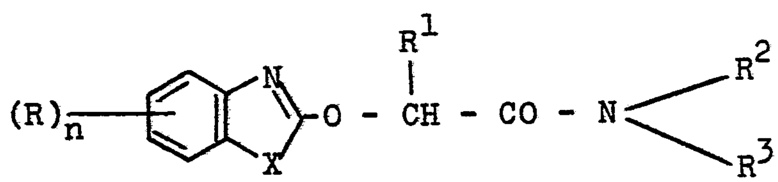

(IV)

In dieser Formel (IV) stehen n für 1, 2, 3 oder 4, jedes R einzeln für Wasserstoff, Halogen, Alkyl, Halogenoalkyl, Alkoxy, Halogenoalkoxy, Alkylthio, Halogenoalkylthio, Amino, Alkylamino, Dialkylamino, Nitro, Cyano oder Alkoxycarbonyl, oder 2 R-Gruppen zusammen genommen für Methylendioxy, Dichloromethylendioxy oder Difluoromethylendioxy,

und $R^1$ für Wasserstoff oder Alkyl; $R^2$ und $R^3$ können gleich oder verschieden sein und jeweils einzeln für Wasserstoff oder Alkyl, Alkenyl, Alkinyl, Aralkyl, Cycloalkyl, Aryl oder Stickstoff-haltige heterocyklische Gruppen stehen, die gegebenenfalls substiuiert sein können, oder sie können zusammen mit dem Stickstoff-Atom, an das sie gebunden sind, eine substituierte oder unsubstituierte, gegebenenfalls teilweise ungesättigte und gegebenenfalls benzo-anellierte monocyklische oder dicyklische Gruppe bilden, die weiterhin ein oder mehrere Hetero-Atome enthalten kann, und X steht für Sauerstoff oder Schwefel.

Als Ergebnis weiterer eingehender Untersuchungen über Verbindungen des vorgenannten Typs substituierter Carbonsäureamide wurde nunmehr gefunden, daß die substituierten Acetanilid-Verbindungen der Formel (I) gemäß der vorliegenden Erfindung eine herbizide Wirkung besitzen, die derjenigen anderer, analoger Verbindungen in unerwartetem Maße überlegen ist.

Die substituierten Acetanilid-Verbindungen der vorstehenden Formel (I) gemäß der vorliegenden Erfindung werden in der oben erwähnten Patentanmeldung nicht beschrieben und besitzen im Vergleich zu den analogen, in dieser Patentanmeldung offenbarten Verbindungen eine in Bezug auf ihre Spezifität in bemerkenswerter Weise hervorragende herbizide Wirkung. Dieses Ergebnis war in keiner Weise vorhersehbar.

Da die Verbindungen gemäß der vorliegenden Erfindung nur eine geringe Toxizität gegenüber warmblütigen Tieren und eine gute Selektivität gegenüber Kulturpflanzen besitzen, d.h. die Eigenschaft, bei den üblicherweise verwendeten Konzentrationen keine Phytotoxizität zu zeigen, können sie vorteilhaft als Herbizide zur Unkrautbekämpfung verwendet

werden. Die Herbizide gemäß der vorliegenden Erfindung zeigen eine überlegene selektive Bekämpfungswirkung, insbesondere wenn sie als Mittel zur Behandlung des Bodens vor der Keimung, sowie als Mittel zur Behandlung der Pflanzen und des Bodens gegen Reisfeld-Unkräuter verwendet werden.

Wie bereits oben festgestellt wurde, weisen die Verbindungen gemäß der vorliegenden Erfindung eine hochgradige Ungefährlichkeit, eine nachgewiesene überlegene herbizide Wirksamkeit und ein breites herbizides Spektrum auf.

Beispielsweise entfalten sie herbizide Wirkung gegen die nachstehend genannten Reisfeld-Unkräuter, ohne eine Phytotoxizität gegenüber Reispflanzen zu zeigen.

Lateinische Bezeichnung der Unkräuter:
Dicotyledonen

Rotala indica Koehne,
Lindernia Procumbens Philcox,
Ludwiga prostrata Roxburgh,
Potamogeton distinctus A. Bennett,
Elatine triandra Schkuhr etc.

Monocotyledonen
Echinochloa cus-galli Beauvois var.,
Monochoria vaginalis Presl,
Eleocharis acicularis L.,
Eleocharis Kuroguwai Ohwi,
Cyperus difformis L.,
Cyperus serotinus Rottboell,
Sagittaria pygmaea Miq,
Alisma canaliculatum A. Braun et Bouche,
Scirpus juncoides Roxburgh var., etc.

Weiterhin entfalten sie eine herbizide Wirkung gegen die nachstehenden Ackerunkräuter, ohne irgendwelche Phytotoxizität gegenüber den weiter unten bezeichneten Kulturpflanzen zu zeigen:

<u>Dicotyledonen</u>
Polygonum sp.,
Chenopodium album Linnaeus,
Stellaria madia Villars,
Portulaca oleracea Linnaeus, etc.

<u>Monocotyledonen</u>
Echinochloa crus-galli Beauv. var.,
Digitaria adscendens Henr.,
Cyperus iria L., etc.

Beispiele für die Kulturpflanzen sind Dicotyledonen wie Senf, Raps, Baumwolle, Möhren, Bohnen, Kartoffeln, Rüben, Kanaris etc. und Monocotyledonen wie Mais, Reis, Hafer,Gerste, Weizen, Hirse, Zuckerrohr etc.

Es wird ausdrücklich erwähnt, daß die vorstehend bezeichneten Pflanzen typische Beispiele für die durch die lateinischen Namen bezeichneten Gattungen sind.

Die Anwendbarkeit der wirksamen Verbindungen der vorliegenden Erfindung ist jedoch nicht auf Unkräuter in Reisfeldern und landwirtschaftlich bebauten Nutzflächen beschränkt, sondern sie sind ebenfalls gegen Unkräuter auf brachliegenden Nutzflächen und dergleichen wirksam. Der hierin verwendete Begriff "Unkräuter" bezeichnet im weitesten Sinne alle Pflanzen, die an Stellen wachsen, an denen sie unerwünscht sind.

Die substituierten Acetanilid-Verbindungen der Formel (I) gemäß der vorliegenden Erfindung können durch das folgende, allgemeine Verfahren synthetisiert werden:

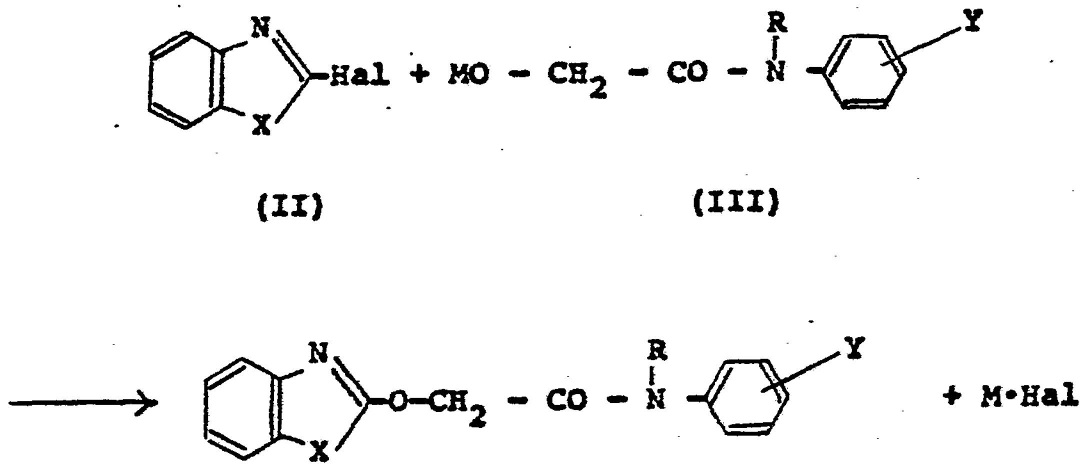

(II) (III)

(I)

(in dem Formelschema haben X, R, Y, Hal und M die vorstehend angegebene Bedeutung).

In dem angegebenen Reaktionsschema sind Beispiele für X ein Sauerstoff-Atom und ein Schwefel-Atom; Beispiele für R sind Alkyl-Gruppen mit 1 - 4 Kohlenstoff-Atomen wie Methyl, Ethyl, n- (oder iso-)Propyl, n- (iso, sec- oder tert-)Butyl etc., und Beispiele für Y sind Alkylthio-Gruppen mit 1 - 4 Kohlenstoff-Atomen, in denen eine Alkyl-Einheit ähnlich der oben genannten vorliegt.

In dem Verfahren zur Herstellung der Verbindungen der Formel (I), das durch das oben gegebene Reaktionsschema veranschaulicht wird, sind Beispiele für das eine Ausgangsmaterial, das 2-Halogenobenzazol der Formel (II),

2-Chlorobenzothiazol,

2-Chlorobenzoxazol etc.,

sowie die entsprechenden 2-Bromo-Verbindungen.

Beispiele für das andere Ausgangsmaterial, die Verbindung der Formel (III), sind

2'-Methylthio-N-methylhydroxyacetanilid,
2'-Ethylthio-N-methylhydroxyacetanilid,
3'-Methylthio-N-methylhydroxyacetanilid,
3'-Propylthio-N-methylhydroxyacetanilid,
4'-Methylthio-N-methylhydroxyacetanilid etc.

sowie deren Alkalimetall-Salze.

Das vorstehende Verfahren wird unter Hinweis auf ein typisches Beispiel näher beschrieben.

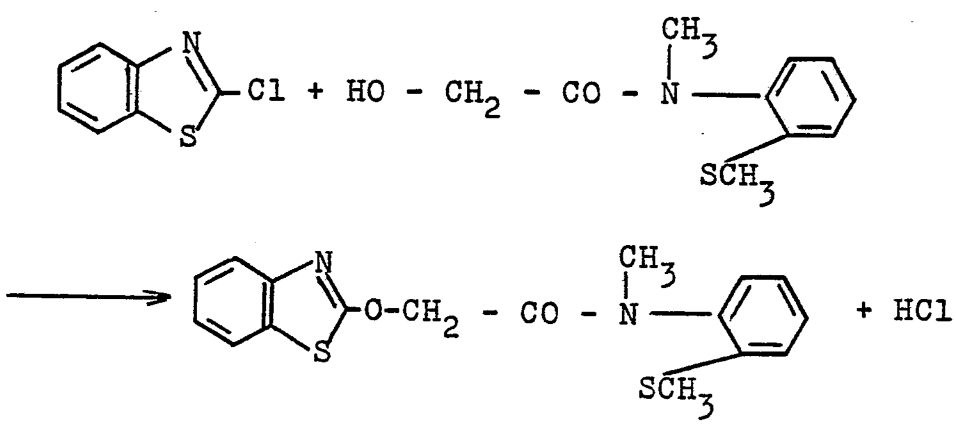

Das Verfahren gemäß der vorliegenden Erfindung kann vorzugsweise in Anwesenheit eines Lösungsmittels oder Verdünnungsmittels durchgeführt werden. Zu diesem Zweck kann jedes inerte Lösungsmittel oder Verdünnungsmittel eingesetzt werden.

Beispiele für solche Lösungsmittel und Verdünnungsmittel sind Wasser; aliphatische, alicyklische und aromatische Kohlenwasserstoffe (die gegebenenfalls chloriert sein

können) wie Hexan, Cyclohexan, Petrolether, Ligroin, Benzol, Toluol, Xylol, Methylenchlorid, Chloroform, Kohlenstoff-Tetrachlorid, Ethylenchlorid, Trichloroethylen, Chlorbenzol etc.; Ether wie Diethylether, Methylethylether, Diisopropylether, Dibutylether,Propylenoxid, Dioxan, Tetrahydrofuran etc.; Ketone wie Aceton, Methylethylketon, Methylisopropylketon, Methylisobutylketon etc.; Nitrile wie Acetonitril, Propionitril, Acrylnitril etc.; Alkohole wie Methanol, Ethanol, Isopropanol, Tert-butanol, Ethylenglycol etc.; Ester wie Ethylacetat, Amylacetat etc.; Säureamide wie Dimethylformamid, Dimethylacetamid etc.; Sulfone und Sulfoxide wie Dimethylsulfoxid, Sulfolan etc.; und organische Basen wie Pyridin etc.

Wie oben erwähnt, kann das Verfahren der vorliegenden Erfindung in Gegenwart eines säurebindenden Mittels durchgeführt werden. Beispiele für solche säurebindenden Mittel sind die allgemein verwendeten Hydroxide, Carbonate, Bicarbonate, Alkoholate etc. der Alkalimetalle, und tertiäre Amine wie Triethylamin, Diethylanilin, Pyridin etc.

Das Verfahren der vorliegenden Erfindung kann in einem weiten Temperaturbereich durchgeführt werden. Im allgemeinen wird es bei einer Temperatur zwischen -20°C und dem Siedepunkt der Reaktionsmischung, vorzugsweise zwischen 0°C und 100°C durchgeführt. Zweckmäßigerweise wird die Reaktion unter atmosphärischem Druck durchgeführt, jedoch kann auch unter erhöhtem oder vermindertem Druck gearbeitet werden.

Für die Verwendung als Herbizide können die Verbindungen der vorliegenden Erfindung mit Wasser verdünnt oder, durch Zusatz landwirtschaftlich unbedenklicher Hilfsstoffe, nach den allgemein auf dem Gebiet der Herstellung von Agrochemikalien angewandten Verfahrensweisen zu verschiedenartigen Präparaten weiterverarbeitet werden. Für die praktische Anwendung können diese verschiedenen Präparate entweder als solche oder nach Verdünnung mit Wasser auf die gewünschte Konzentration verwendet werden.

Die hierin genannten landwirtschaftlich unbedenklichen Hilfsstoffe sind Verdünnungsmittel (Lösungsmittel, Füllstoffe, Träger etc.), oberflächenaktive Mittel (Lösungsvermittler, Emulgatoren, Dispergiermittel, Netzmittel etc.), Stabilisatoren, Haftmittel, Aerosol-Treibgase, synergistische Mittel etc.

Beispiele für die Lösungsmittel sind Wasser und organische Lösungsmittel, beispielsweise Kohlenwasserstoffe [wie n-Hexan, Petrolether, Naphta, Erdölfraktionen (Paraffinwachs, Kerosin, Gasöl, Mittelöl, Schweröl etc.), Benzol, Toluol, Xylol etc.], halogenierte Kohlenwasserstoffe [wie Methylenchlorid, Kohlenstofftetrachlorid, Trichloroethylen, Ethylenchlorid, Ethylendibromid, Chlorbenzol, Chloroform etc.], Alkohole [wie Methylalkohol, Ethylalkohol, Propylalkohol, Ethylenglycol etc.], Ether [wie Diethylether, Ethylenoxid, Dioxan etc.], Alkohol-Ether [wie Ethylenglycol-Monomethylether etc.], Ketone [wie Aceton, Isophoron etc.], Ester [wie Ethylacetat, Amylacetat etc.], Amide [wie Dimethylformamid, Dimethylacetamid etc.], Sulfoxide [wie Dimethylsulfoxid etc.] und dergleichen.

Beispiele für die Füllstoffe oder Träger sind anorganische teilchenförmige Feststoffe wie Schwefel, gelöschter Kalk, Magnesiumkalk, Gips, Calciumcarbonat, Siliciumoxid, Perlit, Bimsstein, Calcit, Diatomeenerde, amorphes Siliciumoxid, Aluminiumoxid, Zeolith, Tonmineralien (wie Pyrophyllit, Talkum, Montmorillonit, Beidellit, Vermiculit, Kaolinit, Glimmer etc.); pflanzliche feinteilige Feststoffe wie

Getreide, Stärke, verarbeitete Stärke, Zucker, Glukose, zerkleinerte Pflanzenstengelerzeugnisse etc.; feinteilige Feststoffe aus synthetischen Harzen wie Phenolharz, Harnstoffharz, Vinylchloridharz etc. und dergleichen.

Beispiele für die oberflächenaktiven Mittel sind anionische oberflächenaktive Mittel wie Alkylsulfate (z.B. Natrium-Laurylsulfat), Arylsulfonate (z.B. Alkylarylsulfonate, Natriumalkylnaphtalinsulfonat etc.), Succinate, Polyethylenglycol-alkylarylether-Schwefelsäureester-Salze etc.; cationische oberflächenaktive Mittel wie Alkylamine (z.B. Laurylamin, Stearyltrimethylammoniumchlorid, Alkyldimethylbenzylammoniumchlorid etc.) und Polyoxyethylenglycolether (z.B. Polyoxyethylenalkylarylether und Kondensationsprodukte aus diesen etc.), Polyoxyethylenglycolester (z.B. Polyoxyethylen-Fettsäureester), und Polyolester (z.B. Polyoxyethylensorbitanmonolaurat);sowie ampholytische oberflächenaktive Mittel.

Beispiele für andere Hilfsstoffe sind Stabilisatoren, Haftmittel (wie landwirtschaftliche Seifen, Kaseinkalk, Natriumalginat, Polyvinylalkohol (PVA), Klebstoffe auf Vinylacetat-Basis, Acryl-Klebstoffe etc.), Aerosol-Treibgase (wie Trichlorofluoromethan, Dichlorofluoromethan, 1,2,2,-Trichloro-1,1,2-trifluoroethan, Chlorbenzol, verflüssigtes Erdgas, niedere Ether etc.), wirksamkeitsverlängernde Mittel, Dispersionsstabilisatoren (wie Casein, Traganth, Carboxylmethylcellulose (CMC), Polyvinylalkohol (PVA) etc.), synergistische Mittel und dergleichen.

Die Verbindungen der vorliegenden Erfindung können mit Hilfe der allgemein auf dem Gebiet der Herstellung von Agrochemikalien angewandten Verfahrensweisen zu verschiedenartigen

Präparaten verarbeitet werden. Derartige Präparate sind beispielsweise emulgierbare Konzentrate, Öl-Lösungen, netzbare Pulver, Lösungen, Suspensionen, Pulver, Granulat, pulvrige Mischungen, Kapseln etc.

Die Zusammensetzungen gemäß der vorliegenden Erfindung können den genannten Wirkstoff in Mengen von 0,001 - 100 Gewichtsprozent, vorzugsweise von 0,005 - 95 Gewichtsprozent enthalten.

Für den praktischen Einsatz liegt die geeignete Menge des Wirkstoffs in den verschiedenen Formulierungen oder gebrauchsfertigen Präparaten im allgemeinen bei 0,01 bis 95 Gewichtsprozent, vorzugsweise bei 0,05 - 60 Gewichtsprozent. Der Gehalt an Wirkstoff kann, je nach der Form des Präparats, dem Verfahren, dem Zweck, der Zeit und dem Ort seiner Anwendung und den Umständen des Unkrautbefalls, in geeigneter Weise variiert werden.

Die Verbindungen der vorliegenden Erfindung können, falls weiter erforderlich, in Kombination mit anderen Pestiziden, wie Insektiziden, Fungiziden, Acariziden, Nematoziden, Viroziden, Herbiziden, Pflanzenwachstums-Regulatoren, Lockstoffen /wie organischen Phosphat-Verbindungen, Carbamat-Verbindungen, Dithio(oder -thiol)carbamat-Verbindungen, organischen Chlorid-Verbindungen, Dinitro-Verbindungen, organischen Schwefel- oder Metallverbindungen, Antibiotika, substituierten Diphenylether-Verbindungen, Harnstoff-Verbindungen und Triazin-Verbindungen/ und/oder Düngemitteln zur Anwendung gebracht werden.

Die verschiedenen, den Wirkstoff gemäß der vorliegenden Erfindung enthaltenden Formulierungen und gebrauchsfertigen Präparate können mit Hilfe der bei der Anwendung von Agrochemikalien allgemein gebräuchlichen Methoden zum Einsatz

gebracht werden, etwa durch Sprühen (z.B. Spritzen, Nebeln, Zerstäuben, Stäuben, Streuen von Granulat, Wasser-Oberflächenanwendung, Gießen etc.), Bodenbehandlung (z.B. Einarbeiten, Sprengen etc.) und dergleichen. Außerdem kann das sogenannte ULV-Verfahren (ultra-low-volume application method) eingesetzt werden, bei dem der Wirkstoff in einer Konzentration von bis zu 100 % vorliegen kann.

Die Dosierung der Formulierung oder des Präparats pro Einheitsfläche beträgt etwa 0,1 - 10 kg/ha, vorzugsweise 0,5 bis 3 kg/ha. In besonderen Fällen ist es jedoch möglich, oder gelegentlich sogar notwendig, eine Dosis außerhalb des vorgenannten Bereichs zu verwenden.

Die vorliegende Erfindung macht herbizide Mittel verfügbar, wie Verbindungen der vorstehenden Formel (I) als Wirkstoffe zusammen mit Verdünnungsmitteln (Lösungsmitteln und/oder Füllstoffen und/oder Trägern) und/oder oberflächenaktiven Mitteln, und falls weiter erforderlich, solchen Hilfsstoffen wie Stabilisatoren, Haftmitteln, synergistischen Mitteln etc. enthalten.

Weiterhin macht die vorliegende Erfindung ein Verfahren zur Unkrautbekämpfung zugänglich, bei dem eine Verbindung der vorstehenden Formel (I) allein oder in Kombination mit einem Verdünnungsmittel (einem Lösungsmittel und/oder einem Füllstoff und/oder einem Träger) und/oder einem oberflächenaktiven Mittel, und falls weiter erforderlich, solchen H ilfsstoffen wie einem Stabilisator, einem Haftmittel, einem synergistischen Mittel etc. auf Unkräuter und/oder deren Lebensraum aufgebracht werden.

Die vorliegende Erfindung wird durch die folgenden Beispiele näher erläutert, sollte jedoch in keiner Weise auf diese

beschränkt werden.

Beispiel 1 (benetzbares Pulver)
15 Teile der Verbindung Nr. 1 der vorliegenden Erfindung, 80 Teile einer 1 : 5-Mischung aus gepulverter Diatomeenerde und gepulvertem Ton, 2 Teile Natrium-Alkylbenzolsulfonat und 3 Teile Natrium-Alkylnaphthalinsulfonat-Formalin-Kondensat wurden gemahlen und miteinander vermischt, wodurch ein benetzbares Pulver gebildet wurde. Dieses wurde mit Wasser verdünnt und durch Sprühen auf Unkräuter und/oder deren Lebensraum aufgebracht.

Beispiel 2 (emulgierbares Konzentrat)
15 Teile der Verbindung Nr. 2 der vorliegenden Erfindung, 70 Teile Methylethylketon, 8 Teile Polyoxyethylenalkylphenylether und 7 Teile Calciumalkylbenzolsulfonat wurden unter Rühren miteinander vermischt, wodurch ein emulgierbares Konzentrat gebildet wurde. Dieses wurde mit Wasser verdünnt und durch Sprühen auf Unkräuter und/oder deren Lebensraum aufgebracht.

Beispiel 3 (Stäubemittel)
2 Teile der Verbindung Nr. 3 der vorliegenden Erfindung und 98 Teile gepulverter Ton wurden vermahlen und miteinander vermischt, wodurch ein Stäubemittel gebildet wurde. Das Stäubemittel wurde auf Unkräuter und/oder deren Lebensraum durch Stäuben aufgebracht.

Beispiel 4 (Stäubemittel)
1,5 Teile der Verbindung Nr. 4 der vorliegenden Erfindung, 0,5 Teile Isopropylhydrogenphosphat (PAP) und 98 Teile gepulverter Ton wurden vermahlen und miteinander vermischt, wodurch ein Stäubemittel gebildet wurde. Dieses wurde auf Unkräuter und/oder deren Lebensraum durch Stäuben aufgebracht.

NIT 141

Beispiel 5 (Granulat)

10 Teile der Verbindung Nr. 5 der vorliegenden Erfindung, 30 Teile Bentonit (Montmorillonit), 58 Teile Talkum und 2 Teile Ligninsulfonat wurden miteinander vermischt und 25 Teile Wasser zu der Mischung gegeben. Die Mischung wurde innig verknetet und mit Hilfe eines Pelletisierextruders fein zerteilt, wodurch ein Granulat mit einer Korngröße von 0,43 - 2,0 mm (10 - 40 mesh) erhalten wurde, das anschließend bei 40°C - 50°C getrocknet wurde. Das erhaltene Granulat wurde auf Unkräuter und/oder deren Lebensraum durch Streuen aufgebracht.

Beispiel 6 (Granulat)

95 Teile Tonteilchen einer Korngrößenverteilung von 0,2 bis 2 mm wurden in einen Drehmischer gegeben, und eine Lösung von 5 Teilen der Verbindung Nr. 6 in einem organischen Lösungsmittel wurde unter Drehen auf die Teilchen aufgesprüht, wodurch diese gleichmäßig benetzt wurden. Die Teilchen wurden dann bei 40°C - 50°C getrocknet, wodurch ein beschichtetes Granulat gebildet wurde. Dieses wurde auf Unkräuter und/oder deren Lebensraum durch Streuen aufgebracht.

Im Vergleich zu aus der Literatur bekannten wirksamen Verbindungen mit analoger Struktur und solchen mit ähnlichem Wirksamkeitstypus sind die wirksamen Verbindungen gemäß der vorliegenden Erfindung durch eine wesentlich verbesserte Wirkung und eine sehr geringe Toxizität gegenüber warmblütigen Tieren gekennzeichnet, und aus diesem Grunde haben die Verbindungen der vorliegenden Erfindung einen hohen Nützlichkeitswert.

Die unerwartete Überlegenheit und bemerkenswerte Wirkung der Wirkstoffe gemäß der vorliegenden Erfindung werden durch

die Ergebnisse der Test-Beispiele aufgezeigt, in denen die speziellen Verbindungen der vorliegenden Erfindung gegen verschiedene Unkräuter eingesetzt wurden.

## Test-Beispiele

**Test der Bekämpfungswirkung gegenüber Reisfeld-Unkräutern unter vollen Bewässerungsbedingungen (Feldversuch):**

### Herstellung des Test-Präparats

Unter Verwendung einer Pelletier-Vorrichtung und eines gebräuchlichen Pelletier-Verfahrens wurde die nachstehende Formulierung hergestellt und zu zylindrischen Pellets von 0,5 mm Durchmesser und 1- 3 cm Länge pelletiert.

### Formulierung (gesamt 100 Teile)

| | |
|---|---|
| Wirkstoff: | 0,8 - 10 Teile, |
| Polyoxyethylenalkylphenylether: | 2,5 Teile, |
| Bentonit: | 30 Teile, |
| Talkum: | 57,5-66,7 Teile; |

die Änderung der Talkum-Menge entspricht der Änderung der Menge des Wirkstoffs, so daß die Summe 100 Teile beträgt.

### Testverfahren

1) Chemische Behandlung vor dem Umpflanzen der Reispflanzen. Das Testfeld wurde entsprechend einem üblichen Reisanbau-Verfahren mit Hilfe eines Traktors auf 20 -25 cm Tiefe gepflügt, und unmittelbar anschließend wurde aus dem Bewässerungskanal Wasser auf das Feld bis zu einer Höhe von 1 - 2 cm geleitet, und die erste Bodenbearbeitungsstufe (tilling) wurde durchgeführt. Einen oder zwei Tage nach der ersten Bodenbearbeitung wurden wiederum Wasser bis zu einer Höhe von 5 cm aufgeleitet und die letzte Bodenbearbeitung durchgeführt. Zwei Tage nach der letzten Bodenbearbeitung wurde das Testfeld unter Verwendung starrer Kunststoffolien

in Parzellen von jeweils 20 $m^2$ (2 m x 10 m) aufgeteilt.

Danach wurde der wie vorstehend formulierte Wirkstoff auf das Wasser aufgebracht. Drei Tage nach der chemischen Behandlung wurden die in einer Pflanzenzuchtanstalt zu diesem Zweck gezogenen Reissetzlinge (Varietät: Kinmaze, 15 Tage alt, 2 - 2,5-Blatt-Stadium, Höhe 15 - 17 cm), und zwar 3 - 5 Pflanzen pro Wurzel, unter Verwendung einer motorbetriebenen Reispflanzmaschine umgepflanzt. Vier Wochen nach der chemischen Behandlung wurde die Unkraut-Bekämpfungswirkung entsprechend dem weiter unten erläuterten Maßstab untersucht.

2) Chemische Behandlung nach dem Umpflanzen der Reispflanzen:
Bis zum Stadium der letzten Bodenbehandlung wurden die bei dem Testverfahren 1). beschriebenen Arbeitsweisen durchgeführt. Zwei Tage nach der letzten Bodenbehandlung wurde die Wassertiefe des Testfelds auf 2 - 3 cm eingestellt, und Reispflanzen wurden unter Einsatz einer motorbetriebenen Reispflanzmaschine in ähnlicher Weise wie im Testverfahren 1) umgepflanzt; unmittelbar anschließend wurde das Feld unter Verwendung starrer Kunststoffolien in Parzellen von jeweils 20 $m^2$ (2m x 10 m) unterteilt, und die Wassertiefe auf dem Feld wurde dann für längere Zeit bei 3 - 5 cm gehalten. Sieben Tage nach dem Umpflanzen der Reispflanzen wurde die wie vorstehend formulierte Verbindung auf das Wasser aufgebracht. Vier Wochen nach der chemischen Anwendung wurde die Bekämpfungswirkung nach dem folgenden Maßstab untersucht.

Die Bewertung der Wirkung wird als prozentuale Unkrautvernichtungsrate, bezogen auf eine unbehandelte Fläche, wie folgt ausgedrückt:

5: 95 % oder mehr (Ausrottung)
4: 80 % oder mehr, jedoch weniger als 95 %
3: 50 % oder mehr, jedoch weniger als 80 %
2: 30 % oder mehr, jedoch weniger als 50 %
1: 10 % oder mehr, jedoch weniger als 30 %
0: weniger als 10 % (keine Wirkung).

Die Bewertung "0" in der Spalte Phytotoxizität gegenüber Reispflanzen bezeichnet die Abwesenheit einer Phytotoxizität.

Die Test-Ergebnisse sind in der nachstehenden Tabelle 1 aufgeführt.

NIT 141

Tabelle 1

| Verbindung Nr. | Wirkstoffmenge kg/ha | Behandlung 3 Tage vor dem Umpflanzen Unkrautbekämpfungswirkung Unkräuter | | | | | | | Phytotoxizität Reis | Behandlung 7 Tage nach dem Umpflanzen Unkrautbekämpfungswirkung Unkräuter | | | | | | | Phytotoxizität Reis |
|---|---|---|---|---|---|---|---|---|---|---|---|---|---|---|---|---|---|
| | | A | B | C | D | E | F | G | | A | B | C | D | E | F | G | |
| 1 | 1,0 | 5 | 5 | 5 | 5 | 5 | 5 | 5 | 0 | 5 | 5 | 5 | 5 | 5 | 5 | 5 | 0 |
| 2 | " | 5 | 5 | 5 | 5 | 5 | 5 | 5 | 0 | 5 | 5 | 5 | 5 | 5 | 5 | 5 | 0 |
| 3 | " | 5 | 5 | 5 | 5 | 5 | 5 | 5 | 0 | 5 | 5 | 5 | 5 | 5 | 5 | 5 | 0 |
| 4 | " | 5 | 5 | 5 | 5 | 5 | 5 | 5 | 0 | 5 | 5 | 5 | 5 | 5 | 5 | 5 | 0 |
| 5 | " | 5 | 5 | 5 | 5 | 5 | 5 | 5 | 0 | 5 | 5 | 5 | 5 | 5 | 5 | 5 | 0 |
| 6 | " | 5 | 5 | 5 | 5 | 5 | 5 | 5 | 0 | 5 | 5 | 5 | 5 | 5 | 5 | 5 | 0 |
| IV-1 | 1,0 | 3 | 4 | 3 | 2 | 0 | 4 | 2 | 0 | 3 | 3 | 3 | 2 | 0 | 4 | 1 | 0 |

Anmerkungen:

1. Die Verbindungen bezeichnenden Zahlen entsprechen denjenigen, die in den nachfolgenden Synthese-Beispielen und der Tabelle 2 verwendet werden.
2. Die Symbole A, B, C, D, E, F und G in der Spalte der Unkräuter bezeichnen die folgenden:

   A: Panicum crus-galli,
   B: Scirpus juncoides Roxburgh var.,
   C: Monochoria vaginalis Presl,
   D: Breitblättrige Unkräuter (Lindernia Procumbens Philcox,
   Rotala indica Koehne, Elatine triandra Schkuhr etc.),
   E: Sagittaria pygmaea Miq.,
   F: Eleocharis acicularis L.,
   G: Cyperus serotinus Rottboell.

3. Die Verbindung Nr. IV-1 ist die durch die folgende Formel bezeichnete:

(IV - 1) 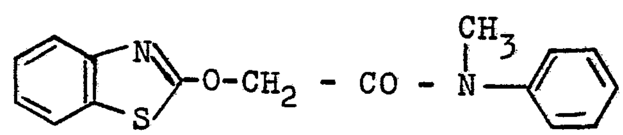

(JP-OS 154762/1979)

Das Verfahren zur Herstellung der Verbindungen gemäß der vorliegenden Erfindung wird durch die folgenden Synthese-Beispiele näher beschrieben.

Synthese-Beispiel 1

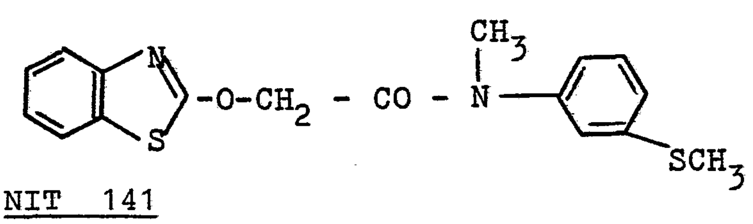 (Verbindung Nr. 1)

NIT 141

4,4 g 85,5%iges Caliumhydroxid wurden in 80 ml Isopropanol aufgelöst, und zu dieser Lösung wurden 11,51 g 3'-Methylthio-N-methylhydroxyacetanilid hinzugefügt, und die Lösung wurde 30 Minuten gerührt. Nach dem Abkühlen auf 0°C wurden tropfenweise 9,8 g 2-Chlorobenzothiazol zugegeben und die Lösung wurde 5 h bei Raumtemperatur gerührt. Nach dem Rühren wurde die Lösung in 200 ml Wasser gegossen, und die entstandenen Kristalle wurden aus Methanol umkristallisiert, wonach 14,4 g des gewünschten Benzothiazol-2-yloxyessigsäure-N-methyl-3-methylthioanilids erhalten wurden; Schmp. 139 bis 140°C.

Synthese-Beispiel 2

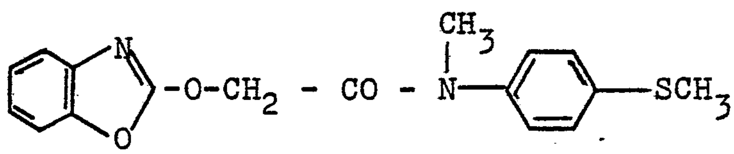

(Verbindung Nr. 2)

0,65 g Calium-tert-butoxid wurden in 10 ml Isopropanol aufgelöst, und zu dieser Lösung wurde 1,0 g 4'-Methylthio-N-methylhydroxyacetanilid hinzugegeben, und anschließend wurde 30 Minuten gerührt. Nach dem Abkühlen auf 0°C wurden tropfenweise 0,77 g 2-Chlorobenzoxazol hinzugefügt, und die Lösung wurde 5 h bei Raumtemperatur gerührt. Nach dem Rühren wurde die Lösung in 50 ml Wasser gegossen und mit Ether extrahiert. Die Ether-Schicht wurde mit Wasser gewaschen, über wasserfreiem Natriumsulfat getrocknet, und das Lösungsmittel wurde abdestilliert. Die entstandenen Kristalle wurden aus Methanol umkristallisiert, wonach 1,2 g des gewünschten Benzoxazol-2-yloxyessigsäure-N-methyl-4-methylthioanilids erhalten wurden; Schmp. 128,5 - 130°C.

In analoger Weise wurden die in Tabelle 2 aufgeführten Verbindungen gemäß der vorliegenden Erfindung hergestellt.

Tabelle 2

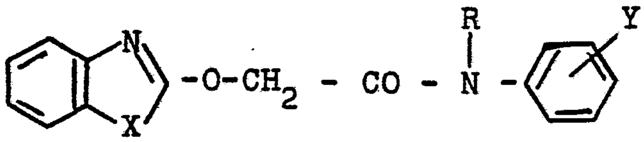

(I)

| Verbindung Nr. | X | R | Y | Schmelzpunkt (°C) |
|---|---|---|---|---|
| 3 | O | $-CH_3$ | $2-S-CH_3$ | 137 – 138 |
| 4 | S | $-CH_3$ | $2-S-CH_3$ | 145 – 146 |
| 5 | O | $-CH_3$ | $3-S-CH_3$ | 95 – 99 |
| 6 | S | $-CH_3$ | $4-S-CH_3$ | 123 – 128 |

## P a t e n t a n s p r ü c h e

1. Substituierte Acetanilid-Verbindungen der Formel

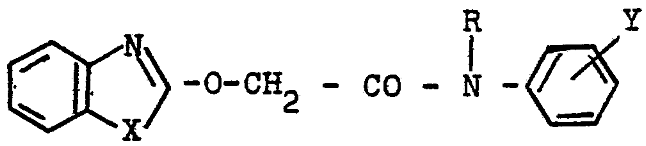

(I)

in der X für ein Sauerstoff-Atom oder ein Schwefel-Atom, Y für eine Alkylthio-Gruppe mit 1 - 4 Kohlenstoff-Atomen und R für eine Alkyl-Gruppe mit 1 - 4 Kohlenstoff-Atomen steht.

2. Verfahren zur Herstellung einer substituierten Acetanilid-Verbindung der Formel

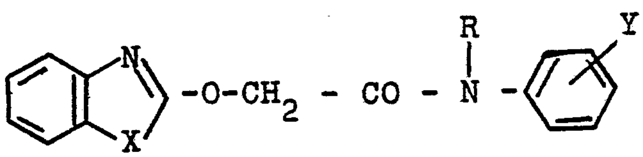

(I)

in der X für ein Sauerstoff-Atom oder ein Schwefel-Atom, Y für eine Alkylthio-Gruppe mit 1 - 4 Kohlenstoff-Atomen und R für eine Alkyl-Gruppe mit 1 - 4 Kohlenstoff-Atomen steht, dadurch gekennzeichnet, daß ein 2-Halogenobenzazol der Formel

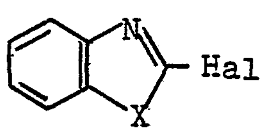

(II)

in der X die vorstehend genannte Bedeutung hat und Hal für ein Halogen-Atom steht, mit einer Verbindung der Formel

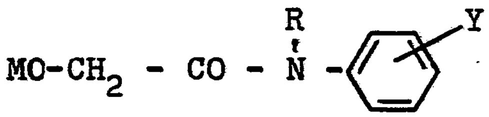 (III)

in der Y und R die vorstehend genannten Bedeutungen haben und M für ein Wasserstoff-Atom oder ein Alkalimetall-Atom steht, umsetzt.

3. Herbizid, enthaltend als Wirkstoff eine substituierte Acetanilid-Verbindung der Formel

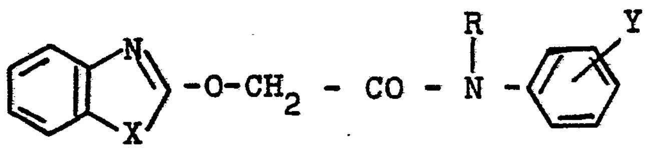 (I)

in der X für ein Sauerstoff-Atom oder ein Schwefel-Atom, Y für eine Alkylthio-Gruppe mit 1 - 4 Kohlenstoff-Atomen und R für eine Alkyl-Gruppe mit 1 - 4 Kohlenstoff-Atomen steht.

4. Verwendung von substituierten Acetaniliden der Formel (I) gemäß Anspruch 1 zur Bekämpfung von Unkraut.

5. Benzothiazol-2-yloxyessigsäure-N-methyl-3-methylthioanilid (Verbindung Nr. 1) gemäß Anspruch 1.

6. Benzoxazol-2-yloxyessigsäure-N-methyl-4-methylthioanilid (Verbindung Nr. 2) gemäß Anspruch 1.

7. Benzoxazol-2-yloxyessigsäure-N-methyl-2-methylthioanilid (Verbindung Nr. 3) gemäß Anspruch 1.

8. Benzothiazol-2-yloxyessigsäure-N-methyl-2-methylthioanilid (Verbindung Nr. 4) gemäß Anspruch 1.

9. Benzoxazol-2-yloxyessigsäure-N-methyl-3-methylthioanilid (Verbindung Nr. 5) gemäß Anspruch 1.

10. Benzothiazol-2-yloxyessigsäure-N-methyl-4-methylthioanilid (Verbindung Nr. 6) gemäß Anspruch 1.

Europäisches Patentamt

**EUROPÄISCHER RECHERCHENBERICHT** 0062254

Nummer der Anmeldung

EP 82 10 2540

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 005 501 (BAYER AG)<br>* Insgesamt *<br>----- | 1-10 | C 07 D 263/58<br>C 07 D 277/68<br>A 01 N 43/74 |

RECHERCHIERTE SACHGEBIETE (Int. Cl. 3)

C 07 D 263/00
C 07 D 277/00
A 01 N 43/00

Der vorliegende Recherchenbericht wurde für alle Patentansprüche erstellt.

| Recherchenort | Abschlußdatum der Recherche | Prüfer |
|---|---|---|
| DEN HAAG | 22-06-1982 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82